# EUROPEAN PATENT APPLICATION

(11) **EP 3 530 131 A1**
(43) Date of publication of application: **28.08.2019**
(21) Application number: 17861857.5
(22) Date of filing: 16.10.2017
(51) Int. Cl.: A41C 5/00, A41H 1/02, A61B 5/107, G01B 21/20

(54) **BRASSIERE PRODUCTION METHOD**

(30) Priority: 20.10.2016 JP 2016206365
(71) Applicant: Shin Nippon Biomedical Laboratories, Ltd., Kagoshima 891-1394 (JP)
(72) Inventor: NAGATA Ryoichi, Kagoshima-shi Kagoshima 891-1394 (JP)
(74) Representative: CSY Herts
(86) International application number: PCT/JP2017/037432
(87) International publication number: WO 2018/074436

(57) **Abstract**

[Problem] To provide a method for producing brassieres that can prevent sagging of breasts, achieve effortless bust uplift, and immobilize the breasts when prone.

[Solution] A brassiere production method comprising a step for determining the shape of a region that comprises a breast (15) of a subject (11) when a nipple (13) of the subject (11) is turned vertically downward. An example of the state in which a nipple (13) is turned vertically downward is a state in which the subject is immobilized on a loading table (21) and, compared to the subject's nipple (13), the subject's back is in the direction of the loading table.

## Description

### TECHNICAL FIELD

The present invention relates to a production method for a brassiere. To explain in more detail, the present invention relates to a production method for a brassiere that is matched to the shape of the breast of the subject wearer when the subject is in a prone state.

### BACKGROUND ART

Made-to-order brassieres that are manufactured to match the shape of a woman's bust are per se known. For example, a three dimensional brassiere cup and a method of producing it are disclosed in Japanese Laid-Open Patent Publication 2015-105460.

### CITATION LIST

### PATENT LITERATURE

Patent Document 1: Japanese Laid-Open Patent Publication 2015-105460.

### SUMMARY OF INVENTION

### TECHNICAL PROBLEM

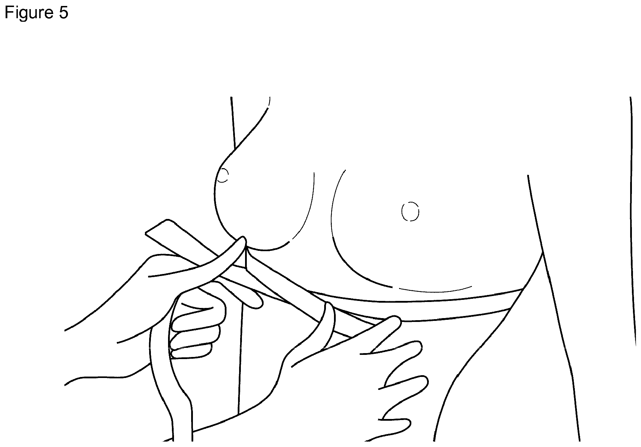

Fig. 5 is a conceptual figure showing the way in which an order-made brassiere is measured. Generally, during the manufacture of an order-made brassiere, the cup is determined in consideration of the under-bust measurement and the top-bust measurement of the subject, and on the basis of the difference between the magnitudes of these factors. For a normal woman, an order-made brassiere is measured with her spine in the extended state. When this is done, the woman's breast comes to be measured in the state in which it is drooping downward due to its own weight. Accordingly, the order-made brassiere comes to be manufactured on the basis of the woman's bust shape in the state in which her spine is extended, or in the state in which her breast is drooping downward due to its own weight, and this implies that the brassiere is corrected so that her breast sags downward more than otherwise.

Furthermore, for cancer patients, sometimes it may happen that radiation treatment or particle beam treatment is performed with the subject in the lateral recumbent posture or in the prone posture (i.e. in the prone state). In this case there is the problem that, if the subject's breast is not held stationary, when the posture of the subject is changed, the radiation etc. may undesirably irradiate her breast.

Accordingly, it is an object of the present invention to provide a method of producing a brassiere, which is capable of preventing sagging downward of the subject's breast, and which implements uplifting of the subject's bust within a reasonable range.

Another object of the present invention is to provide a method of producing a brassiere, which is capable of fixating the subject's breast when she is in the prone state.

### SOLUTION TO TECHNICAL PROBLEM

The present invention is based upon the realization that it is possible to manufacture an order-made brassiere by ascertaining the shape of the bust of the subject, which includes her breast, in the state in which her nipple is oriented in a downward direction, i.e. based upon the shape of her breast in a state in which it is not sagging downward.

Furthermore, the present invention is based upon the realization that since, during radiation treatment for cancer, sometimes it is the case that irradiation with radiation etc. is performed when the subject is not in a supine posture but rather is in a lateral recumbent posture or a prone posture, accordingly, if an order-made brassiere is used that is based upon the shape of her breast in the prone state, then it is possible effectively to fixate her breast even when she is in a lateral recumbent posture or a prone posture.

The present invention relates to a production method for a brassiere. This method includes a process of ascertaining the shape of a site upon a subject 11 including a breast 15, in a state in which a nipple 13 of the subject 11 is oriented in a vertically downward direction.

An example of a state in which the nipple 13 is oriented in a vertically downward direction is a state in which the subject is fixed to a loading table 21, and, as compared to the nipple of the subject, the back of the subject is directed toward the direction of the loading table.

An example of the process of ascertaining the shape of the region including the breast 15 of the subject 11 may include a process of measuring the top-bust and the under-bust of the breast. At this time, it would also be possible to arrange to measure the distance from the under-bust to the nipple.

The process of ascertaining the shape of the region including the breast 15 of the subject 11 may also include a process of photographing the region including the breast of the subject with a three dimensional scanner.

This method may be termed a method for production of a brassiere on the basis of the shape of the breast of the subject when the subject is in a prone state.

It should be understood that the present invention also provides a production method for a brassiere that includes a process of photographing a region including a breast of a subject with a three dimensional scanner. For example, in the case of a cancer patient the size of whose bust is small, not very much movement of the breast takes place even if her posture changes. In a case of this type, if it is possible to manufacture a brassiere that fits the shape of her breast, then it is possible to manufacture a brassiere that can fixate her breast in an appropriate manner even if her posture varies.

### ADVANTAGEOUS EFFECTS OF INVENTION

The present invention can provide a method of producing a brassiere, which is capable of preventing sagging downward of the subject's breast, and which implements uplifting of her bust within a reasonable range.

Moreover, the present invention can provide a method of producing a brassiere, which is capable of fixating the subject's breast when she is in the prone state.

### BRIEF DESCRIPTION OF DRAWINGS

Fig. 1 is a conceptual figure showing a state in which a nipple of a subject is oriented in a vertically downward direction;
Fig. 2 is a conceptual figure showing an example of a measurement device for an order-made brassiere;
Fig. 3 is a conceptual figure showing a situation when the subject is mounted upon a loading table;
Fig. 4 is a conceptual figure for explanation of a holding device; and
Fig. 5 is a conceptual figure showing a situation in which measurement is being performed.

### DESCRIPTION OF EMBODIMENTS

In the following, an embodiment of the present invention will be explained with reference to the drawings. However, the present invention is not to be considered as being limited to the embodiment described below; the present invention also includes in its scope other variant embodiments that would be obvious to a person of ordinary skill in the art on the basis of the following disclosure.

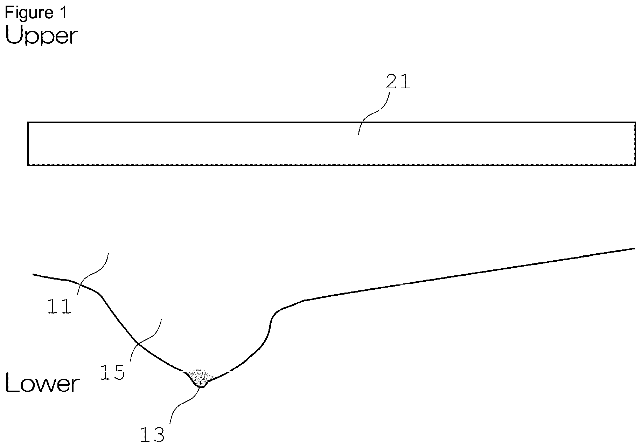

The present invention relates to a production method for a brassiere. A brassiere is normally an item of woman's haberdashery (underwear) for holding sites including both her breasts. The basic configuration of a brassiere itself is per se known, and typically includes portions that cover the two breasts and a portion that links them together behind the subject's body. The present method of producing a brassiere includes a process of ascertaining the shape of a site upon a subject 11 including her breast 15, in a state in which a nipple 13 of the subject 11 is oriented in a vertically downward direction. Fig. 1 is a conceptual figure showing a state in which the nipple of the subject is oriented in a vertically downward direction. In this example, the subject 11 is a woman. Another example of a subject 11 would be a woman suffering from cancer. And the subject 11 may also be a man whose pectoral region is large.

In the state in which the nipple 13 is oriented in a vertically downward direction, the nipple 13 need not necessarily be in a vertically downward orientation, provided that the nipple 13 is pointing in a generally downward direction. Specifically, a state in which the nipple 13 is in a vertically downward direction may simply be a state in which the nipple is lower than the subject's back (for example, a state in which the subject is prone, and the breast is not in a state of being pressed or deformed). An example of a state in which the nipple 13 is oriented in a vertically downward direction is a state in which the subject is fixed to a loading table 21 and the back of the subject is positioned more directed toward the direction of the loading table as compared to the nipple 13 of the subject, and the subject's nipple is positioned lower than her back. In concrete terms, the subject 11 is fixed supine upon the loading table 21. At this time one, or two or more, of the head, the shoulders, the pectoral region, the abdomen, the arms, and the legs of the subject 11 may be fixed to the loading table 21 by fixing means (i.e. belts or the like). Next, in this state in which the subject 11 is fixed to the loading table 21, the loading table is tilted or rotated through half a turn, so that a state is established in which the subject 11 is tilted (a prone position) or is in a prone state (i.e. a state in which her back is securely fixed to the loading table 21, while the front of her body is left free). In this manner, a state is reached in which the subject is fixed to the loading table 21, and the back of the subject is positioned more directed toward the direction of the loading table as compared to her nipple 13. A rotating gantry for cancer treatment is an example of such equipment.

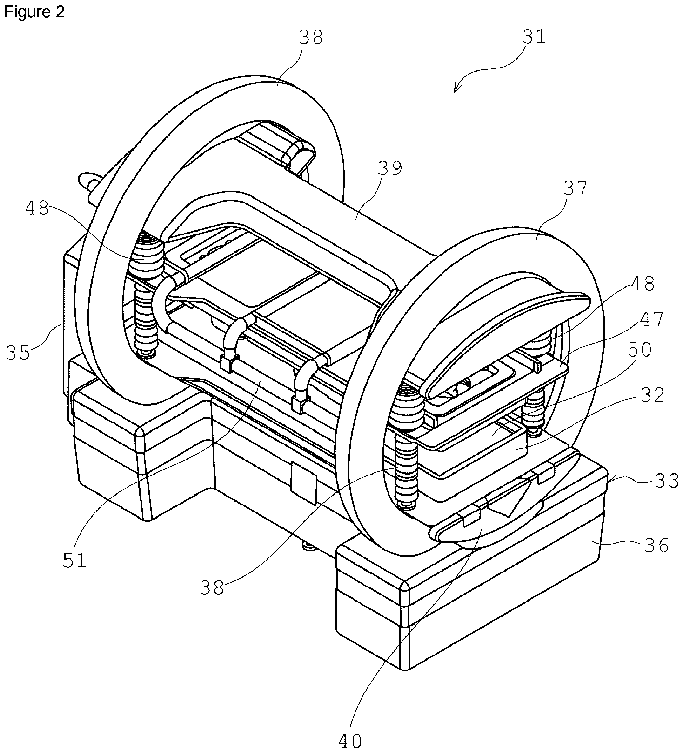

Fig. 2 is a conceptual figure showing an example of a measurement device for an order-made brassiere. As shown in Fig. 2, this device 31 comprises a loading table 21 (32) for fixing the subject as mounted in a required posture, a main body 33 for supporting the loading table 32, and a drive control unit 35 for controlling the operation of an electric motor or the like not shown in the figures for rotating the loading table 32. The loading table 32 and the main body 33 may also be provided separately. The main body 33 includes a base 36 that functions as a floor stand, a front annular portion 37 and a rear annular portion 38 that incorporate a rotation mechanism for rotating the loading table 32, an upper beam 39, and a lower beam 40 for holding the loading table 32. The drive control unit 35 incorporates a control unit that controls the accuracy of the loading table 31 and also incorporates a control unit that raises and lowers a pressure plate 47 and controls the vertical position of the pressure plate 47. Elongated ball screws 48 are attached to each of the four corners of the upper beam 39 and the lower beam 40. In the example shown in the figure, these ball screws are covered with bellows pipes. It is arranged for the pressure plate 47 to be shifted upward or downward when the ball screws 48 are rotated. An elastic member 50 is provided under the bottom plate of the loading table 32. And a reinforcement frame 51 is attached to the upper surface of the pressure plate 47. In the example shown in the figure, parts of this reinforcement frame 51 come down to the left and right of the pressure plate 17.

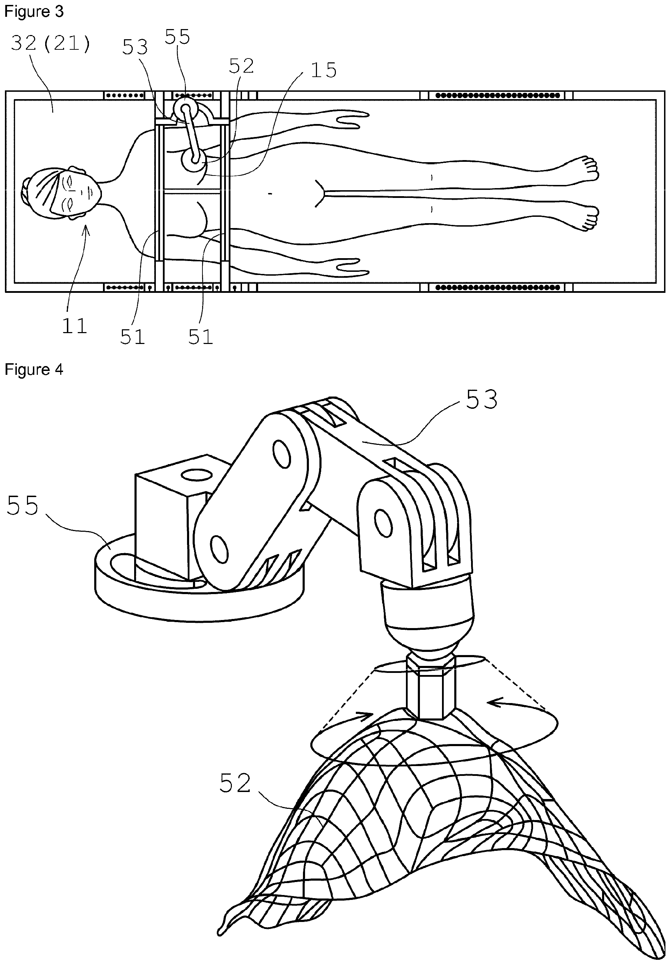

Fig. 3 is a conceptual figure showing the way in which a subject is mounted upon the loading table. The subject 11 climbs onto the upper portion of the loading table 32 and lies supine. In this state, the subject is fixed to the loading table 32 with belts or the like not shown in the figures. Then the loading table 32 is slid along the upper surface of the lower beam 40, so that the subject is conveyed from the front of the front annular portion in the inward direction, and is shifted as far as the rear annular portion 30. Next, the drive control unit 35 drives the ball screws 38, and shifts the pressure plate 47 to an appropriate position where it can loosely press the subject. And next, the drive control unit 35 rotates the loading table 32 by driving its motor, and, due to this, the subject can be rotated. This rotation may be around a rotational direction similar to the interiors of the front annular portion 37 and the rear annular portion 38 (with respect to an axis from the subject's head to her feet, around a rotational direction taking that axis as center, or around a rotational direction taking an axis parallel to that axis as center). With the subject in an appropriate position (for example, in a state in which the nipple of the subject is pointing in the vertically downward direction), the shape of a region that includes the breast 15 of the subject 11 is ascertained. The method of ascertaining the shape of this site that includes the breast 15 of the subject 11 (i.e. her pectoral region) may be CT-scanning or the like.

In the example shown in Fig. 3, a holding device is provided for holding the subject's breast. Fig. 4 is a conceptual figure for explanation of this holding device. This holding device 52 is attached to the loading table 32 or to the reinforcement frame 51 via an arm portion 53 and a fixing lug 55. The arm portion 53 is capable of performing rotation, and extension and retraction. Moreover, the fixing lug may be capable of shifting with respect to the reinforcement frame 51. Furthermore, instead of the holding device 51, it would also be possible to provide a photographic unit (for example a camera). In this case, by photographing the pectoral region of the subject while moving, the photographic unit would be able to reproduce a stereoscopic image of the pectoral region of the subject.

An example of the process of ascertaining the shape of a region including the breast 15 of the subject 11 could also include a process of measuring her top-bust and her under-bust. In this case, while the subject is suspended in a prone state in midair, an appropriate person may perform measurement of dimensions required for manufacturing a brassiere, such as measurement of the top-bust and the under-bust of the subject 11, and so on.

The process of ascertaining the shape of a region including the breast 15 of the subject 11 may also include a process of photographing a region that includes the breast of the subject. This photography may be performed by three dimensional scanning or X-ray photography or the like; and it would also be possible to capture images from a plurality of locations, and, by combining these captured images, to reproduce a region including the breast of the subject three dimensionally. An example of a three dimensional scanner is a CT scanner. By employing a photographic technique such as CT scanning or the like, it is possible to ascertain the shape of the bust of the subject three dimensionally. By doing this, for example, the shape of the subject's nipple may be ascertained, and a brassiere may be manufactured that reflects the shape of the nipple. In this case, a brassiere that is manufactured by this method not only has a cavity that reflects the shape of the subject's breast, but also has a hollow portion that reflects the shape of her nipple, and these hollow portions fit both the nipple and the breast as a combination. Since this brassiere has this type of shape, accordingly it can effectively prevent the breast wobbling. Moreover, by performing CT scanning, it is also possible simultaneously to diagnose whether or not the subject is suffering from breast cancer. Furthermore, if the positions of the shape of the bust of the subject and of its breast cancer can be ascertained in three dimensions, then it is also possible to perform sampling of the tissue of the subject while her breast is held fixed. Yet further, it would also be possible to manufacture a brassiere having a hole portion for collecting target tissue, or a brassiere with a specified location for irradiation with radiation or the like, or a brassiere on which a portion where radiation or the like is to be irradiated is formed with a hole portion. Even further, it is possible to create a three dimensional model of the subject's pectoral region by employing the three dimensional data for the pectoral region obtained by CT scanning. If this is done, it is possible to create a sample of a brassiere and to fit this sample to the three dimensional model. Moreover, a treatment plan may be formulated by using the three dimensional model.

This method may be termed a method for production of a brassiere on the basis of the shape of the breast of the subject when the subject is in the prone state. Naturally, this method may also be employed for underwear for medical use, for example for underwear for corrective use and so on. A brassiere that is manufactured on the basis of the present invention can hold the subject's breast in a preferred state, and it is envisaged that the excellent fit that it provides can also be effective for improving symptoms of various types of disorders, including psychiatric disorders. However, of course the production method of the present invention is not intended to be employed by the doctor as a treatment, or for diagnosis; it is preferred that the production method of the present invention is employed for manufacturing a brassiere to be used as haberdashery (i.e. underwear).

As described above, after having ascertained the shape of the pectoral region of the subject or her bust shape, the brassiere may be manufactured according to a per se known method. If the shape of the region on the subject including her breast has been acquired electronically, then the brassiere may be manufactured according to this shape information that has thus been acquired. A manufacturing device for a brassiere of this type may be controlled by a computer. Such a computer should comprise an input/output unit, a control unit, a calculation unit, and a storage unit. Each of these elements is capable of transmitting and receiving information. And information required for manufacturing a brassiere with the appropriate raw material, shape, pattern and design, color, positions of any hole portions, and so on is stored in the storage unit. The information required for manufacturing the brassiere described above is inputted to the computer. This input may also be provided by the subject 11 selecting the desired information while it is being displayed upon a monitor. The data such as the shape of the pectoral region of the subject 11 and so on that has been photographed by CT scanning is inputted to the computer. When this has been done, the control unit reads out the required control program from the storage unit, and, on the basis of commands in this control program, performs the required calculation processing with the calculation unit by employing the data that has been inputted, such as the shape of the subject's pectoral region and so on, and the required information for manufacturing the brassiere. In this manner, the computer obtains the information required for manufacturing the brassiere, including the necessary dimensional data, and stores it in the storage unit in an appropriate manner. By doing this, design drawings may be obtained that include the dimensions of the brassiere. An implementer may manufacture the brassiere according to these design drawings that have thus been obtained. Moreover, by the computer transmitting the required data that has been obtained to an automatic loom or the like, it would also be possible for the brassiere to be manufactured by the loom.

The present invention also provides a method of producing a brassiere that includes a process of photographing a region including the breast of the subject with a three dimensional scanner. For example, with a cancer patient the size of whose bust is small, not very much movement of the breast takes place even if her posture changes. In a case of this type, if it is possible to manufacture a brassiere that fits the shape of her breast, then it is possible to manufacture a brassiere that can fixate her breast in an appropriate manner even if her posture varies.

### POSSIBILITY OF INDUSTRIAL APPLICATION

The present invention may be employed in the field of haberdashery.

### REFERENCE SIGNS LIST

11: subject
13: nipple
15: breast
21: loading table

## Claims

1. A production method for a brassiere, including a process of ascertaining the shape of a site upon a subject (11) including a breast (15) in a state in which a nipple (13) of the subject (11) is oriented in a vertically downward direction.

2. A production method for a brassiere according to Claim 1, wherein said state in which said nipple (13) is oriented in a vertically downward direction is the state in which said subject is fixed to a loading table (21), and, as compared to said nipple (13) of said subject, the back of said subject is directed toward the direction of said loading table.

3. A production method for a brassiere according to Claim 1, wherein
the process of ascertaining the shape of a region including said breast (15) of said subject (11) includes a process of measuring the top-bust and the under-bust of said breast.

4. A production method for a brassiere according to Claim 1, wherein the process of ascertaining the shape of a region including said breast (15) of said subject (11) includes a process of photographing said region including said breast of said subject with a three dimensional scanner.

5. A production method for a brassiere according to Claim 1, on the basis of the shape of the breast of said subject when said subject is in a prone state.

6. A production method for a brassiere, comprising a process of photographing a region including a breast of a subject with a three dimensional scanner.
